(19) Europäisches Patentamt
European Patent Office
Office européen des brevets

(11) EP 0 636 360 B1

(12) **FASCICULE DE BREVET EUROPEEN**

(45) Date de publication et mention de la délivrance du brevet:
**17.06.1998 Bulletin 1998/25**

(51) Int. Cl.[6]: **A61K 7/48**, A61K 7/42, A61K 7/13

(21) Numéro de dépôt: **94401508.0**

(22) Date de dépôt: **01.07.1994**

(54) **Composition cosmétique contenant en association une superoxyde dismutase et un pigment mélanique**

Kosmetisches Präparat mit einer Zusammensetzung einer Superoxyd Dismutase und eines Melaninpigmentes

Cosmetic composition containing an association of a superoxide dismutase and a melanin pigment

(84) Etats contractants désignés:
**CH DE ES FR GB IT LI**

(30) Priorité: **02.07.1993 FR 9308155**

(43) Date de publication de la demande:
**01.02.1995 Bulletin 1995/05**

(73) Titulaire: **L'OREAL**
**75008 Paris (FR)**

(72) Inventeurs:
- **Colin, Christian**
  **F-75020 Paris (FR)**
- **N'Guyen, Quang Lan**
  **F-92160 Antony (FR)**

(74) Mandataire:
**Tonnellier, Jean-Claude**
**Nony & Associés,**
**29, rue Cambacérès**
**75008 Paris (FR)**

(56) Documents cités:
**FR-A- 2 287 899**

EP 0 636 360 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des compositions cosmétiques, hygiéniques ou pharmaceutiques contenant une superoxyde-dismutase (SOD) en association avec des pigments mélaniques.

De telles compositions utilisées par voie topique permettent notamment de lutter contre le vieillissement cutané et de protéger la peau contre les effets des radicaux libres induits par exemple par les polluants atmosphériques et/ou par le rayonnement ultra-violet. On sait en effet que la toxicité des polluants atmosphériques, notamment des polluants gazeux comme le dioxyde de soufre, l'ozone et les oxydes d'azote, est liée à leur activité d'initiateurs de radicaux libres qui sont la source de phénomènes d'oxydation provoquant chez les êtres vivants des dommages cellulaires. Les cellules d'organes qui sont en contact direct et permanent avec le milieu extérieur, comme la peau, le cuir chevelu et certaines muqueuses, sont particulièrement sensibles à ces effets des polluants gazeux, qui se traduisent notamment par un vieillissement accéléré de la peau, avec un teint manquant d'éclat et une formation précoce de rides et de ridules, et aussi par une diminution de la vigueur et un aspect terne des cheveux.

On sait que les superoxyde-dismutases sont des enzymes capables d'induire la dismutation des ions superoxydes, suivant la réaction :

$$2O\bullet^-_2 + 2H^+ \xrightarrow{SOD} H_2O_2 + O_2$$

On connaît de nombreuses superoxyde-dismutases.

Par exemple, on a déjà décrit des superoxyde-dismutases extraites d'érythrocytes de boeuf (Markovitz, J. Biol. Chem. 234, p. 40, 1959) et des superoxyde-dismutases extraites d'Escherichia coli (Keele et Fridovich, J. Biol. Chem., 245, p. 6176, 1970). Dans la demande de brevet français n° 73.13670 déposée le 16 Avril 1973 sont décrites des superoxyde-dismutases extraites de souches bactériennes marines, ainsi que leur procédé de préparation.

Les superoxyde-dismutases permettent notamment de protéger la peau et les cheveux notamment en maintenant l'intégrité de la structure kératinique naturelle; voir par exemple la demande de brevet FR-A-2287899. En outre, les superoxyde-dismutases améliorent la respiration cellulaire cutanée et maintiennent ou améliorent les qualités de la peau telles que la douceur au toucher, la souplesse et l'élasticité. Leur présence dans des compositions pour cheveux permet aussi le maintien ou l'amélioration de l'état du cuir chevelu, tout en protégeant également la peau des mains de la personne qui applique ces compositions.

Les superoxyde-dismutases protègent aussi la peau contre les phénomènes d'inflammation causés par les rayonnements ultra-violets ainsi que contre le vieillissement accéléré de la peau notamment sous l'influence de tels rayonnements.

Grâce à ces différentes propriétés, les superoxyde-dismutases sont utilisables dans des compositions cosmétiques pour la peau, les cheveux ou les muqueuses, ainsi que dans des compositions pharmaceutiques à usage dermatologique.

Le mécanisme d'action des superoxyde-dismutases peut être considéré au moins en partie comme un effet antiradicaux libres. L'ion superoxyde $O\bullet^-_2$ (oxygène actif) est un ion-radical dont l'instabilité et la réactivité en font un composé toxique, car il engendre,notamment en présence d'ions métalliques, des radicaux libres hydroxyle ($OH\bullet$) hautement nocifs. La SOD exerce un effet protecteur notamment en piégeant les ions superoxydes et constitue donc un système biologique de défense contre les effets nocifs des radicaux libres.

Par ailleurs, on sait que certains pigments mélaniques ont déjà été utilisés dans des compositions cosmétiques pour la protection de l'épiderme humain contre les UV, le maquillage de la peau, des cils et des sourcils ou encore la coloration des cheveux.

Il a maintenant été découvert que, de façon surprenante, l'association d'une superoxyde-dismutase (SOD) avec des pigments mélaniques permet de renforcer d'une manière synergique l'action antiradicalaire exercée par la SOD.

L'invention a tout particulièrement pour objet une composition cosmétique ou dermopharmaceutique caractérisée par le fait qu'elle comprend au moins une superoxyde-dismutase en association avec au moins un pigment mélanique. Les pigments mélaniques sont présents à raison de 0,001 à 1% en poids dans ladite composition.

Par l'expression "SOD" on entend les enzymes SOD et tout produit équivalent ayant une activité analogue à celle des superoxyde-dismutases, à savoir toute enzyme naturelle qui peut catalyser la réaction de dismutation ci-dessus indiquée ainsi que tout produit ayant cette activité, ce qui inclut notamment les enzymes modifiées telles que les SOD modifiées par greffage de polyoxyalkylènes, de polyéthylèneglycols, de polysaccharides ou de groupes acylés, ainsi que les substances contenant de tels produits. On peut citer à cet égard la demande de brevet européen n° EP 223.257.

Les enzymes SOD, qui sont des métalloprotéines dont le constituant métallique est le fer, le manganèse, le cuivre et/ou le zinc, peuvent être d'origines diverses.

On peut notamment citer les SOD d'origine animale (par exemple bovine ou porcine), humaine (par exemple placentaire ou sanguine), végétales (par exemple extraites de champignons, algues, épinards,...), ou extraites de micro-organismes (bactéries ou levures), ou encore une SOD recombinante obtenue par génie génétique.

Parmi les exemples de SOD d'origine bovine, on peut citer en particulier la SOD de type Cu-Zn qui a été purifiée jusqu'à homogénéité et approuvée pour des applications cliniques (New Trends in Allergy, I. Ring et al, Ed. Springer Verlag 1986).

Parmi les exemples de SOD obtenues à partir de cultures de bactéries, levures ou cellules animales, recombinantes on peut citer la SOD Cu-Zn humaine recombinante commercialisée par la Société UBE Industries Ltd.

Parmi les exemples de SOD extraites de bactéries, on peut citer en particulier celles extraites d'Escherichia coli ; parmi les superoxyde-dismutases extraites de champignons, on peut citer en particulier celles extraites de Pleurotus olearius ; parmi les superoxyde-dismutases extraites du sang, on peut citer les érythrocupréines.

On peut citer également les superoxyde-dismutases extraites de souches bactériennes marines, telles que par exemple des souches de Photobacterium phosphoreum, Photobacterium leiognathi ou Photobacterium sepia.

Parmi les diverses souches utilisables, on peut citer les souches de Photobacterium phosphoreum n° ATCC 11040, de Photobacterium leiognathi n° ATCC 25521, de Photobacterium sépia n° ATCC 15709, d'Escherichia coli n° ATCC 15224 et de Pleurotus olearius Gillet (Laboratoire de Cryptogamie de Paris).

Les SOD utilisées selon l'invention peuvent être préparées par application des méthodes déjà décrites par exemple dans l'article de KEELE et al (supra) ainsi que dans Eur. J. Rheumatol. and Inflammation, 4, 173-182 (1982).

Les superoxyde-dismutases extraites de souches bactériennes marines peuvent être préparées selon le procédé décrit dans la demande de brevet français n° 73.13670 ci-dessus mentionnée.

La SOD utilisée selon l'invention peut aussi être une SOD modifiée notamment selon l'enseignement de l'"International Conference on Medical, Biochemical and Chemical Aspects of Free Radicals" (1988 Kyoto) p. 317, article de MORIMOTO H., ou selon l'article de M. YUKIO Ando p. 318 (même source), ou encore selon les documents JP-01250304 (Kanebo) et JP 02273176. On peut citer également la SOD modifiée décrite dans les demandes de brevet européen EP 424 033 et EP 426 488.

Les SOD utilisées selon l'invention peuvent être en outre employées sous forme stabilisée selon les techniques connues, par exemple à l'aide de phosphate, en présence de chlorure de métal alcalin et de saccharose ; voir par exemple le document FR-2.634.125.

Les pigments mélaniques utilisés dans les compositions de l'invention sont insolubles dans les solvants usuels, y compris les solvants aqueux.

Les pigments mélaniques utilisables selon l'invention sont en particulier :

- les pigments mélaniques dérivés de sources naturelles ou synthétiques et qui peuvent être obtenus: (A) par oxydation d'au moins un composé indolique ou indolinique, ou (B) par polymérisation, oxydante ou enzymatique, de précurseurs mélaniques, ou (C) par extraction de la mélanine à partir de substances en contenant, ou (D) par culture de microorganismes.

(A) Les pigments mélaniques peuvent, en premier lieu, être obtenus par oxydation d'au moins un composé indolique choisi notamment parmi ceux répondant à la formule :

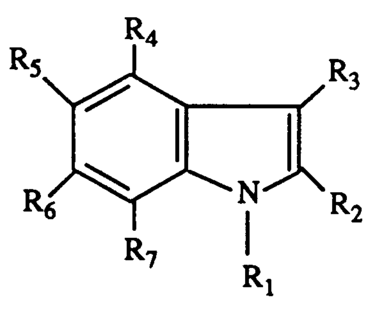

(I)

dans laquelle :

- $R_1$ et $R_3$ représentent, indépendamment l'un de l'autre, un atome d'hydrogène ou un groupe alkyle en $C_1$-$C_4$;
- $R_2$ représente un atome d'hydrogène, un groupe alkyle en $C_1$-$C_4$, un groupe carboxyle ou un groupe alcoxy ($C_1$-$C_4$)-carbonyle ;
- les substituants $R_4$ à $R_7$ représentent chacun indépendamment un atome d'hydrogène, un groupement alkyle en $C_1$-$C_4$, un groupement -NHR° ou -OZ, R° désignant un atome d'hydrogène, un groupe acyle en $C_2$-$C_4$ ou hydroxyalkyle en $C_2$-$C_4$, Z désignant un atome d'hydrogène, un groupe acyle en $C_2$-$C_{14}$, un groupe alkyle en $C_1$-

$C_4$, ou un groupe triméthylsilyle,

- et $R_5$ peut en outre représenter un atome d'halogène,
  étant entendu que :
- au moins l'un des substituants $R_4$ à $R_7$ représente un groupement OZ ou NHR°, l'un au plus des substituants $R_4$ à $R_7$ représentant NHR° et deux au plus des substituants $R_4$ à $R_7$ représentant OZ et, dans ce dernier cas, lorsque Z représente un atome d'hydrogène, les deux substituants représentant OH sont $R_5$ et $R_6$,
- et au moins l'un des substituants $R_4$ à $R_7$ représente un atome d'hydrogène, et dans le cas où un seul de ces substituants représente un atome d'hydrogène, un seul parmi les substituants $R_4$ à $R_7$ représente alors NHR° ou OZ, et les autres substituants $R_4$ à $R_7$ représentent alors un groupe alkyle en $C_1$-$C_4$, ou encore, le cas échéant, pour $R_5$, un atome d'halogène ;

ainsi que les esters ou les sels de ces composés. Parmi ces sels, on citera en particulier les sels d'addition tels que les chlorhydrates, les bromhydrates, les sulfates et méthanesulfonates, ainsi que les sels dérivant des composés pour lesquels $R_2$ est un groupe carboxylique, tels que les sels de métaux alcalins ou alcalino-terreux, d'ammonium ou d'amines. Les esters sont par exemple les esters phosphoriques.

Les composés indoliques de formule (I) ci-dessus sont, par exemple, parmi le 4-hydroxyindole, 5-hydroxyindole, le 6-hydroxyindole, le 7-hydroxyindole, le 4-hydroxy-5-méthoxyindole, le 4-hydroxy-5-éthoxyindole, le 2-carboxy-5-hydroxyindole, le 5-hydroxy-6-méthoxyindole, le 6-hydroxy-7-méthoxyindole, le 5-méthoxy-6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthyl indole, le 4-hydroxy 5-éthoxy N-méthyl indole, le 6-hydroxy 5-méthoxy 2-méthyl indole, le 6-hydroxy 5-méthoxy 2,3-diméthyl indole, le 6-hydroxy 2-éthoxycarbonyl indole, le 7-hydroxy 3-méthyl indole, le 5-hydroxy 6-méthoxy 2,3-dimethyl indole, le 5-hydroxy 3-méthyl indole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 7-aminoindole, le N-méthyl 6-hydroxyéthylaminoindole, le 6-amino 2,3-diméthylindole, le 6-amino 2,3,4,5-tétraméthyl indole, le 6-amino 2, 3, 4-triméthylindole, le 6-amino 2,3,5-triméthylindole, le 6-amino 2,3,6-triméthylindole, le 5,6 diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.

Le 5,6-dihydroxyindole est l'un des composés (I) préférés.

Les pigments mélaniques peuvent être également obtenus par oxydation d'au moins un composé indolinique choisi notamment parmi ceux répondant à la formule :

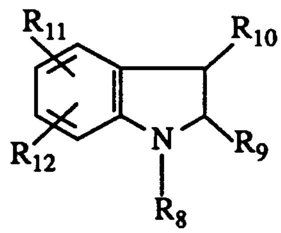 (II)

dans laquelle :

- $R_{10}$ et $R_8$ représentent, indépendamment l'un de l'autre, un atome d'hydrogène ou un radical alkyle en $C_1$-$C_4$,
- $R_9$ représente un atome d'hydrogène, un radical alkyle en $C_1$-$C_4$, un groupe carboxyle ou alcoxy ($C_1$-$C_4$) carbonyle,
- $R_{12}$ désigne un atome d'hydrogène, un radical alkyle en $C_1$-$C_4$, hydroxyle, alcoxy ($C_1$-$C_4$), amino ou alkylamino en $C_1$-$C_{10}$, ou halogène,
- $R_{11}$ désigne un atome d'hydrogène, un groupe hydroxyle, alcoxy en $C_1$-$C_4$, ou amino,
  avec la condition qu'au moins un des radicaux $R_{11}$ et $R_{12}$ désigne un groupe hydroxyle, alcoxy ou amino, et avec la condition que, lorsque $R_{11}$ désigne un groupe amino, $R_{12}$ ne peut pas désigner un radical alkylamino,
- $R_{11}$ et $R_{12}$ pouvant également former un groupement alkylènedioxy en $C_1$-$C_2$, lorsqu'ils sont en positions 5 et 6,
  et leurs sels ou esters.

Les composés répondant à la formule (II) sont choisis notamment dans le groupe constitué par la 5,6-dihydroxyindoline, la 6-hydroxyindoline, la 5,6-méthylènedioxyindoline, la 7-méthoxy 6-hydroxyindoline, la 6,7-dihydroxyindoline, la

5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Dans les composés de formule (II), les radicaux alkyle en $C_1$-$C_4$ désignent de préférence méthyle, éthyle, propyle, isopropyle, butyle, isobutyle ; les radicaux en $C_1$-$C_{10}$ désignent de préférence méthyle, éthyle, propyle, isopropyle, butyle, isobutyle, 1-méthylhexyle, 1-méthylheptyle, 1-méthyloctyle ; les radicaux alcoxy désignent, de préférence, méthoxy, éthoxy, propoxy ou butoxy; halogène désigne, de préférence, brome, chlore ou iode.

Les sels des composés de formule (II) sont, en particulier, des chlorhydrates, bromhydrates, sulfates, méthanesulfonates ou des sels de métaux alcalins, alcalino-terreux, d'ammonium ou d'amines.

L'oxydation du composé indolique de formule (I) ou indolinique de formule (II) peut être effectuée en milieu aqueux ou eau-solvant(s), à l'air, éventuellement en présence d'un agent alcalin et/ou d'un catalyseur métallique d'oxydation tel que par exemple l'ion cuivrique.

Le milieu réactionnel est, de préférence, constitué par de l'eau et peut, le cas échéant, être constitué par un mélange d'eau et d'au moins un solvant choisi de telle façon qu'il solubilise rapidement le composé indolique ou indolinique. Parmi ces solvants, on peut citer, à titre d'exemples, les alcools inférieurs en $C_1$-$C_4$, tels que l'alcool éthylique, l'alcool propylique ou isopropylique, l'alcool tert-butylique, les alkylèneglycols tels que l'éthylèneglycol, le propylèneglycol, les alkyléthers d'alkylèneglycols, tels que les éthers monométhylique, monoéthylique et monobutylique de l'éthylèneglycol, les monométhyléthers du propylèneglycol et du dipropylèneglycol, et le lactate de méthyle.

L'oxydation peut également être effectuée à l'aide de peroxyde d'hydrogène notamment en présence d'un agent alcalin, tel que, par exemple, l'ammoniaque, ou en présence d'ions iodure, l'iodure étant (en particulier) l'iodure d'un métal alcalin, alcalino-terreux ou d'ammonium.

On peut également procéder à l'oxydation à l'aide d'autres oxydants tels que l'acide périodique et ses sels hydrosolubles et dérivés, les permanganates et bichromates, par exemple, de sodium ou de potassium, l'hypochlorite de sodium, le ferricyanure de potassium, le persulfate d'ammonium, un oxyde d'argent ou de plomb, le chlorure ferrique, le nitrite de sodium, les sels de terres rares de degré d'oxydation élevé, dont, notamment le cérium, et des oxydants organiques choisis parmi les ortho- et para-benzoquinones, les ortho-et para-benzoquinones mono- ou di-imines, les 1,2- et 1,4- naphtoquinones, les 1,2-et 1,4-naphtoquinones mono- ou di-imines telles que définies, par exemple, dans la demande EP-A-0 376 776. L'un des sels d'acide periodique préférés est le periodate de sodium.

Il est possible d'activer les agents oxydants par un modificateur de pH.

On peut également effectuer une oxydation enzymatique.

Dans tous les cas, il se forme un produit d'oxydation insoluble. Le produit insoluble peut être isolé par filtration, centrifugation, lyophilisation ou atomisation, et ensuite broyé ou micronisé pour atteindre la granulométrie désirée.

(B) Les pigments mélaniques peuvent également provenir de la polymérisation oxydante ou enzymatique de précurseurs mélaniques, tels que la L-tyrosine, la L-dopa, le catéchol et leurs dérivés.

(C) Les pigments mélaniques peuvent aussi provenir de l'extraction, selon les méthodes connues, de la mélanine de substances naturelles telles que les cheveux humains ou l'encre de céphalopodes (seiches, poulpes), encore connue sous le nom de sépiomélanine. Ces pigments peuvent être purifiés et broyés avant utilisation.

(D) Les pigments mélaniques peuvent en outre être obtenus par culture de microorganismes produisant de la mélanine soit naturellement, soit par modification génétique. Des modes de préparations de tels pigments sont décrits par exemple dans la demande de brevet WO-90 04029.

Le pigment mélanique peut aussi se présenter sous la forme d'un pigment composite. Le pigment mélanique peut ainsi être présent à la surface d'une charge particulaire, ou incorporé dans une charge particulaire,minérale ou organique, lamellaire ou non lamellaire, colorée ou non. Par exemple, le pigment composite peut résulter de l'oxydation d'au moins un composé (I) ou (II), en mélange avec la charge particulaire, dans un milieu essentiellement non-solvant pour ladite charge, ou encore résulter de la polymérisation oxydante du précurseur mélanique sur une charge particulaire.

La charge particulaire peut être toute charge particulaire utilisée ou utilisable dans des compositions cosmétiques, et ayant par exemple des dimensions inférieures à 100 $\mu$m.

De telles charges particulaires sont par exemple des particules minérales non lamellaires.

Les particules minérales non lamellaires utilisées dans ce procédé sont en particulier des particules minérales inertes ayant une granulométrie inférieure à 20 micromètres. De telles particules sont notamment des particules de carbonate de calcium, de silice ou d'oxyde de titane.

De tels pigments mélaniques composites, déposés sur charges minérales, ainsi que leur préparation, sont décrits

notamment dans la demande de brevet FR-2.618.069.

Par un procédé analogue, on peut préparer des pigments mélaniques composites avec des particules minérales colorées compatibles avec une utilisation dans des produits cosmétiques. On appelle ici "particules minérales colorées" des particules non blanches, notamment des particules constituées de sels métalliques, insolubles dans le milieu cosmétique, telles que celles référencées dans le Color Index sous le chapitre "Inorganic Colouring Matters" et portant les numéros 77000 à 77947, autres que les pigments blancs.

Les particules organiques non lamellaires utilisables dans la réalisation des pigments composites sont des particules de polymères naturels ou synthétiques, organiques ou inorganiques, compatibles avec une utilisation dans les cosmétiques, ayant par exemple un poids moléculaire compris entre 5.000 et 5.000.000. Des pigments mélaniques composites sur de telles particules polymères, ainsi que leur préparation, sont décrits dans la demande de brevet européen n° 379.409.

Les particules lamellaires utilisables dans la réalisation de pigments composites sont des particules minérales ou organiques se présentant sous forme de feuillets éventuellement stratifiés. Ces feuillets se caractérisent par une épaisseur plus faible que la plus grande dimension des particules. Par exemple, le rapport entre la plus grande dimension et l'épaisseur est compris entre 2 et 100. La dimension la plus grande est généralement inférieure à 50 micromètres. De tels pigments mélaniques composites déposés sur charge lamellaire sont décrits, ainsi que leur préparation, dans la demande de brevet européen n° 467.767.

L'association synergique de l'invention trouve principalement une application dans la réalisation de compositions cosmétiques ou dermopharmaceutiques destinées à lutter contre le vieillissement cutané et/ou à protéger la peau, les cheveux ou les muqueuses contre les effets nocifs et/ou inesthétiques provoqués par les radicaux libres induits notamment par les polluants atmosphériques et/ou par le rayonnement ultra-violet. L'invention a donc également pour objet de telles compositions.

Dans les compositions de l'invention, la concentration de la SOD peut être par exemple dans la gamme de 40 à 5000 unités enzymatiques de SOD pour 100 g de composition, et notamment de 300 à 1500 unités. L'unité enzymatique de SOD est définie par Mc Cord et Fridovitch, J. Biol. Chem. *244*, 6049 (1969).

Les pigments mélaniques peuvent être présents notamment à raison de 0,05 à 0,5 % en poids.

Les compositions selon l'invention peuvent renfermer la SOD et les pigments mélaniques soit à titre d'ingrédient actif principal, soit à titre de protecteur contre l'oxydation des autres ingrédients. Dans le cas où l'ingrédient oxydable à protéger subit une décomposition accélérée en présence des fibres kératiniques et/ou de la peau et/ou des muqueuses, la SOD avec le pigment mélanique peuvent être conservés seuls, en solution aqueuse diluée ou concentrée, ou sous forme de complexe ou de lyophilisat, et n'être ajoutés aux autres ingrédients de la composition qu'au moment de l'emploi.

De même, lorsque la SOD et le pigment mélanique sont utilisés dans le but de maintenir ou d'améliorer les qualités de la peau ou des cheveux, ou des muqueuses, ces substances peuvent n'être ajoutées à la composition qu'au moment de l'emploi.

Les compositions selon l'invention peuvent donc se présenter sous la forme d'un conditionnement en plusieurs parties contenant d'une part la SOD avec le pigment mélanique, et d'autre part les autres ingrédients de la composition. Comme indiqué ci-dessus, la SOD et le pigment mélanique peuvent être conservés par exemple sous forme de solution aqueuse, de complexe ou de lyophilisat.

Les compositions pour la peau selon l'invention peuvent contenir, outre l'association binaire SOD et pigments mélaniques, des ingrédients actifs ou excipients utilisés de façon usuelle dans les formulations cosmétiques ou dermopharmaceutiques, tels que des tensio-actifs, des colorants, des parfums, des agents conservateurs, des émulsionnants, des véhicules liquides tels que l'eau, des corps gras destinés à constituer la phase grasse d'émulsions (telles que laits ou crèmes), des résines, etc. Les composés destinés à constituer une phase grasse sont par exemple des huiles minérales ou organiques, végétales ou synthétiques, des cires, des alcools gras ou encore des acides gras.

Parmi les huiles minérales, on peut citer par exemple l'huile de vaseline, et parmi les huiles synthétiques, les palmitates d'éthyle et d'isopropyle, les myristates d'alkyle tels que le myristate d'isopropyle, de butyle, ou de cétyle, le stéarate d'hexyle, les triglycérides des acides octanoïque et décanoïque (par exemple le produit vendu sous la dénomination "MIGLYOL" par la Société DYNAMIT-NOBEL), le ricinoléate de cétyle, l'octanoate de stéaryle (huile de purcellin) et l'octanoate de polyisobutène hydroxylé.

Parmi les huiles végétales, on peut citer par exemple l'huile d'amande douce, l'huile d'avocat, l'huile de coco, l'huile de germe de blé, l'huile de maïs, l'huile de ricin, l'huile d'olive, l'huile de palme, l'huile de sésame, l'huile de soja, l'huile d'argan, l'huile d'onagre, l'huile de bourrache, les huiles essentielles et les cires végétales telles que la cire d'abeille ou encore les cires synthétiques telles que les cires de silicone.

Parmi les alcools gras, on peut citer l'alcool cétylique, l'alcool stéarylique, l'alcool myristique, l'alcool hydroxystéalylique, l'alcool oléique, l'alcool isostéarylique, l'alcool laurylique, l'alcool hexadécylique, l'alcool ricinoleylique, l'alcool béhénylique, l'alcool érucylique et le 2-octyldodécanol.

Parmi les acides gras, on peut mentionner l'acide stéarique, l'acide myristique, l'acide palmitique, l'acide oléique,

l'acide linoléique, l'acide laurique, l'acide isostéarique, l'acide hydroxystéarique, l'acide linolénique, l'acide ricinoléique, l'acide arachidique, l'acide behénique, l'acide érucique et les acides lanoliniques.

Les compositions selon l'invention destinées à une application topique sont notamment des solutions ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou émulsions de consistance molle du type crème ou gel, ou encore des microgranulés, ou des dispersions vésiculaires de type ionique et/ou non ionique.

Les compositions de l'invention sont plus particulièrement des compositions conditionnées, c'est-à-dire des compositions disposées dans un conteneur approprié qui est lui-même éventuellement disposé dans un emballage individuel.

Ces compositions sont préparées selon les méthodes usuelles. Elles constituent notamment des crèmes de nettoyage, de protection ou de soin pour le visage, pour les mains ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, ou des compositions désodorisantes contenant un agent bactéricide.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Les compositions pour cheveux selon l'invention peuvent être présentées sous forme de solutions aqueuses, alcooliques ou hydroalcooliques, ou sous forme de crèmes, de gels, d'émulsions, de mousses ou encore sous forme de composition pour aérosol contenant également un agent propulseur sous pression.

Outre les ingrédients actifs classiques, elles peuvent renfermer divers adjuvants habituellement présents dans ces compositions pour cheveux par exemple des véhicules liquides ou sous forme de gels, des parfums, des colorants, des agents conservateurs, des agents épaississants, etc.

L'association synergique selon l'invention peut être incorporée, à titre d'ingrédient principal ou secondaire, dans diverses compositions pour soins capillaires constituant par exemple des crèmes, lotions, gels, sérums ou mousses pour le soin du cuir chevelu, des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent être notamment :

- des shampooings contenant, outre une superoxyde dismutase et les pigments mélaniques, un détergent cationique, anionique ou non ionique,
- des compositions de teinture, y compris des shampooings colorants, qui contiennent des colorants ou des précurseurs de colorant usuels,
- des compositions pour le premier temps (temps de réduction) d'une déformation des cheveux, contenant des dérivés réducteurs tels que mercaptans, sulfites, etc.,
- des compositions pour le ralentissement de la chute des cheveux et pour favoriser la repousse des cheveux, contenant des composés tels que le "Minoxidil" (2,4-diamino-6-pipéndino-pyrimidine-3-oxyde) et ses dérivés, le Diazoxide (7-chloro-3-méthyl-1,2,4-benzothiadiazine 1,1-dioxyde) et le "Phenytoïn" (5,5-diphényl imidazolidine 2,4-dione).

Les compositions de l'invention sont notamment des compositions exemptes d'agent oxydant, et en particulier exemptes de peroxyde d'hydrogène.

La composition cosmétique de l'invention peut aussi être à usage bucco-dentaire, par exemple une pâte dentifrice. Dans ce cas, la composition peut contenir des adjuvants et additifs usuels pour les compositions à usage buccal et notamment des agents tensio-actifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Les compositions cosmétiques selon l'invention peuvent être aussi bien des compositions prêtes à l'emploi que des concentrés devant être dilués avant l'utilisation. Les compositions pouvant être présentées sous forme de concentrés sont par exemple des shampooings ou des compositions pour bains.

La présente invention a en outre pour objet un procédé de traitement cosmétique caractérisé par le fait que l'on applique sur la peau, sur les cheveux, ou sur les muqueuses, une composition telle que décrite ci-dessus contenant au moins une SOD en association avec au moins un pigment mélanique.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple: application de crèmes, de gels, de sérums, de lotions, de laits de démaquillage ou de compositions anti-solaires sur la peau ou sur les cheveux, application d'une lotion pour cheveux sur cheveux mouillés, shampooings, ou encore application de dentifrice sur les gencives.

Le procédé de traitement cosmétique de l'invention est mis en oeuvre de façon à appliquer une quantité efficace de la SOD et de pigment mélanique, c'est à dire une quantité suffisante pour obtenir l'effet de protection recherché.

Ce procédé de traitement cosmétique est destiné en particulier à maintenir la structure kératinique de la peau ou des cheveux, de façon à éviter la dégradation de ceux-ci et les effets inesthétiques d'une telle dégradation sous l'influence des radicaux libres induits notamment par les polluants atmosphériques, à maintenir ou améliorer les qualités de la peau (douceur, souplesse, élasticité), des cheveux ou des muqueuses, à protéger la peau ou les cheveux contre les effets nocifs des rayons ultra-violets, et en particulier à traiter ou prévenir le vieillissement précoce de la peau.

L'invention a également pour objet l'utilisation, en association à effet synergique, d'au moins une SOD et d'au moins un pigment mélanique, tels que définis ci-dessus, comme ingrédients actifs dans la préparation d'une composition cosmétique destinée à protéger la peau ou les cheveux contre les effets des radicaux libres et/ou à traiter ou prévenir le vieillissement précoce de la peau.

Les exemples suivants illustrent l'invention. Dans ces exemples, les proportions indiquées sont des pourcentages en poids.

Exemples de formulation :

**EXEMPLE 1** : ***Emulsion Huile dans eau***

| | |
|---|---|
| SOD (vendue par la Société PENTAPHARM et titrant 3,13 U/mg), q.s.p. 600 unités | 0,19 g |
| Pigment mélanique obtenu par polymérisation oxydante du 5,6-dihydroxyindole en présence d'eau oxygénée et d'ammoniaques | 0,05 g |
| Polyéthylèneglycol polyoxyéthyléné à 50 moles d'oxyde d'éthylène | 1,5 g |
| Monostéarate de diglycéryle | 1,5 g |
| Huile de vaseline | 24 g |
| Alcool cétylique | 2,5 g |
| Triéthanolamine q.s. pH 7 | |
| Eau q.s.p | 100 g |

Cette émulsion, préparée de manière usuelle, peut être utilisée notamment comme crème de jour.

**EXEMPLE 2 : *Emulsion Eau dans l'huile***

| | |
|---|---|
| SOD (vendue par la Société BIO-TECHNOLOGIE et titrant 8037 u/mg), q.s.p. 1000 unités | 0,00012 g |
| Pigment mélanique (selon exemple 1) | 0,1 g |
| Pigment (oxydes de fer) | 0,5 g |
| Sesquiisostéarate de polyglycéryle | 4 g |
| Cire d'abeille blanche | 0,5 g |
| Stéarate de magnésium | 1,5 g |
| Stéarate d'aluminium | 1 g |
| Huile de ricin hydrogénée polyoxyéthylénée (à 7 moles d'oxyde d'éthylène) | 3 g |

(suite)

| | |
|---|---|
| Palmitate d'isopropyle | 10 g |
| Perhydrosqualène | 15 g |
| Eau q.s.p. | 100 g |

Cette émulsion, préparée de manière usuelle, est utilisable notamment comme crème de soin.

**EXEMPLE 3 :** ***Dispersion vésiculaire***

| | |
|---|---|
| Amphiphile non ionique* | 0,9 g |
| Acylglutamate de sodium HS21 (AJINOMOTO) | 0,1 g |
| Glycérine | 3 g |
| SOD (selon exemple 1) | 0,08 g |
| Pigment mélanique obtenu par polymérisation oxydante du 5,6-dihydroxyindole en présence d'eau oxygénée et d'ammoniaque | 0,1 g |
| Pigments (oxyde de fer) | 0,5 g |
| Perhydrosqualène | 10 g |
| Parahydroxybenzoate de méthyle | 0,2 g |
| Acide polyacrylique réticulé (Carbopol 940-Société GOODRICH) | 0,4 g |
| Triéthanolamine q.s. pH = 7 | |
| Eau q.s.p. | 100 g |

* L'amphiphile non ionique est un mélange de produits répondant à la formule suivante :

$$C_{12}H_{25}\text{-}[OC_2H_3(R)\text{-}O\text{-}C_3H_5(OH)\text{-}O]_n\text{-}H$$

dans laquelle :

n, représentant le nombre statistique de motifs, est égal à 2,7, les groupements $-OC_2H_3(R)-$ représentent des radicaux :

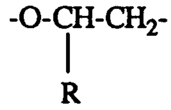 et 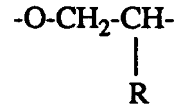

et les groupements $-C_3H_5(OH)-O-$ représentent des radicaux :

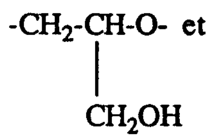 et 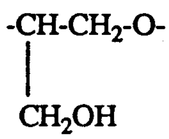

et les groupements R représentent les radicaux $C_{14}H_{29}$ et $C_{16}H_{33}$, statistiquement en quantités molaires égales.

Le produit vendu sous la dénomination "Acylglutamate HS21" est un stéarylglutamate disodique.

Cette dispersion vésiculaire est préparée de la façon suivante:

- le composé amphiphile non ionique est ajouté au cholestérol et à l'acylglutamate à une température de 100°C;
- la température est abaissée à 90°C et on ajoute à cette température la glycérine, la mélanine, les pigments et l'eau;
- le mélange est refroidi à 50°C, la SOD ajoutée, puis on homogénéise 2 fois 4 minutes à l'aide d'un homogénéiseur Virtis 60 (à 40.000 RPM);
- le produit obtenu est refroidi rapidement jusqu'à la température ambiante et dilué avec 20 g d'eau. La phase huileuse (perhydrosqualène et parahydroxybenzoate de méthyle) est ajoutée puis on homogénéise 2 fois 4 minutes à 40.000 RPM;
- le gel de Carbopol (Carbopol 940 et eau q.s.p. 100 g) est dispersé pendant 30 secondes à 10.000 RPM puis l'ensemble est neutralisé par la triéthanolamine.

On obtient une crème lisse et brillante, utilisable notamment comme crème de soin.

Test d'inihibition de la production d'éthylène :

Dans une boîte de Petri de diamètre 32 mm, sont introduits dans l'ordre suivant :

- 1,4 ml de tampon phosphate 50 mM (pH = 7,4),
- 100 µl de solution 200 mM de méthionine,
- 100 µl de solution 4 mM de chlorure ferrique
- 100 µl du produit à tester,
- 100 µl de solution 4 mM d'E.D.T.A.,
- 100 µl de solution 400 mM de N.A.D.H.,
- 100 µl de solution 2 mM de riboflavine.

Cette boîte est ensuite placée sur une coupelle en aluminium et recouverte d'une cellule en quartz afin d'être exposée sous les U.V.A. (365 nm) à la dose d'1 $J/cm^2$.

L'ensemble composé de N.A.D.H.,de riboflavine, de chlorure ferrique et d'E.D.T.A., soumis à l'exposition des U.V.A., va générer des espèces réduites de l'oxygène : $O_2^{-}\bullet$, $H_2O_2$, et principalement le radical hydroxyle $OH\bullet$.

Ce dernier va réagir avec la méthionine en libérant de l'éthylène dont la quantité est mesurée par chromatographie

en phase gazeuse.

Les substances étudiées sont introduites à l'aide d'une micropipette. Ce sont :

- la SOD (origine : PENTAPHARM titrant 3,13 U/mg),
- le pigment mélanique : obtenu par polymérisation oxydante du 5,6-dihydroxyindole on présence d'eau oxygénée et d'ammoniaque.

Conditions chromatographiques : (appareil de type VARIAN 3740)

- température injecteur : 80°C,
- température colonne : 80°C,
- température détecteur : 250°C,
- pression d'hélium : 36 psi (soit environ $2,4.10^5$ Pa),
- colonne : alumina F1 60/80 mesh (origine: SUPELCO),
- longueur : 2 m,
- diamètre externe : 1/8.

Les résultats (moyenne de 3 essais) exprimés en pourcentage de diminution de la production d'éthylène par rapport au témoin (contenant 100 µl de tampon phosphate remplaçant le produit à tester), sont résumés dans le tableau suivant :

TABLEAU DE RESULTATS

| ECHANTILLON | ETHYLENE % d'inhibition |
|---|---|
| Témoin | |
| SOD 0,10 % | 59,68 ± 7,53 |
| Pigment mélanique 0,10 % | 31,14 ± 6,21 |
| SOD + pigment mélanique 0,05 % + 0,05 % | 70,78 ± 4,92 |

L'étude de ces résultats par l'analyse de variance à 1 facteur montre qu'ils sont statistiquement significatifs.

Les pourcentages indiqués sont en poids. Une teneur de 0,10 % en poids de SOD correspond dans le cas présent à 313 unités de SOD pour 100 g de composition.

**Revendications**

**1.** Composition cosmétique, hygiénique ou dermopharmaceutique, caractérisée par le fait qu'elle comprend, en association, au moins une superoxyde dismutase et au moins un pigment mélanique, ledit pigment mélanique étant présent à raison de 0,001 à 1% en poids.

**2.** Composition selon la revendication 1, caractérisée par le fait que ladite superoxyde dismutase est choisie parmi les enzymes naturelles capables de catalyser la réaction de dismutation des ions superoxydes, et tout produit équivalent ayant une telle activité.

**3.** Composition selon l'une quelconque des revendications précédentes, caractérisée par le fait que ledit pigment mélanique est choisi parmi :

- les produits d'oxydation d'au moins un composé indolique ou indolinique ;
- les produits de polymérisation de précurseurs mélaniques ;
- les produits résultant de l'extraction de substances naturelles contenant de la mélanine ;
- et les pigments mélaniques obtenus par culture de microorganismes.

**4.** Composition selon la revendication précédente, caractérisée par le fait que ledit composé indolique répond à la formule :

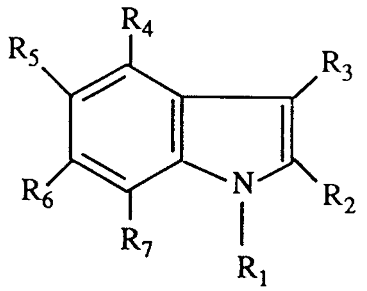

(I)

dans laquelle :

- $R_1$ et $R_3$ représentent, indépendamment l'un de l'autre, un atome d'hydrogène ou un groupe alkyle en $C_1$-$C_4$ ;
- $R_2$ représente un atome d'hydrogène, un groupe alkyle en $C_1$-$C_4$, un groupe carboxyle ou un groupe alcoxy ($C_1$-$C_4$)-carbonyle ;
- les substituants $R_4$ à $R_7$ représentent chacun indépendamment un atome d'hydrogène, un groupement alkyle en $C_1$-$C_4$, un groupement -NHR° ou -OZ, R° désignant un atome d'hydrogène, un groupe acyle en $C_2$-$C_4$ ou un groupe hydroxyalkyle en $C_2$-$C_4$ et Z désignant un-atome d'hydrogène, un groupe acyle en $C_2$-$C_{14}$, un groupe alkyle en $C_1$-$C_4$, ou un groupe triméthylsilyle,
- et $R_5$ peut en outre représenter un atome d'halogène,
  étant entendu que :
- au moins l'un des substituants $R_4$ à $R_7$ représente un groupement OZ ou NHR°, l'un au plus des substituants $R_4$ à $R_7$ représentant NHR° et deux au plus des substituants $R_4$ à $R_7$ représentant OZ et, dans ce dernier cas, lorsque Z représente un atome d'hydrogène, les deux substituants représentant OH sont $R_5$ et $R_6$,
- et au moins l'un des substituants $R_4$ à $R_7$ représente un atome d'hydrogène, et dans le cas où un seul de ces substituants représente un atome d'hydrogène, un seul parmi les substituants $R_4$ à $R_7$ représente alors NHR° ou OZ, et les autres substituants $R_4$ à $R_7$ représentent alors un groupe alkyle en $C_1$-$C_4$, ou encore, le cas échéant, pour $R_5$, un atome d'halogène ;

  ainsi que les esters ou les sels de ces composés.

5. Composition selon la revendication précédente, caractérisée par le fait que ledit pigment mélanique est obtenu par oxydation du 5,6-dihydroxyindole.

6. Composition selon l'une quelconque des revendications 1 à 3, caractérisée par le fait que ledit composé indolinique répond à la formule :

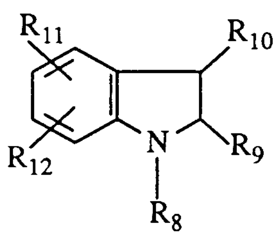

(II)

dans laquelle :

- $R_{10}$ et $R_8$ représentent, indépendamment l'un de l'autre, un atome d'hydrogène ou un radical alkyle en $C_1$-$C_4$,
- $R_9$ représente un atome d'hydrogène, un radical alkyle en $C_1$-$C_4$, un groupe carboxyle ou alcoxy ($C_1$-$C_4$) carbonyle,
- $R_{12}$ désigne un atome d'hydrogène, un radical alkyle en $C_1$-$C_4$, hydroxyle, alcoxy ($C_1$-$C_4$), amino ou alkylamino en $C_1$-$C_{10}$, ou halogène,
- $R_{11}$ désigne un atome d'hydrogène, un groupe hydroxyle,

  avec la condition qu'au moins un des radicaux $R_{11}$ et $R_{12}$ désigne un groupe hydroxyle, alcoxy ou

amino, et avec la condition que, lorsque $R_{11}$ désigne un groupe amino, $R_{12}$ ne peut pas désigner un radical alkylamino,

- $R_{11}$ et $R_{12}$ pouvant également former un groupement alkylènedioxy en $C_1$-$C_2$, lorsqu'ils sont en positions 5 et 6,

et leurs sels ou esters.

7. Composition selon la revendication 3, caractérisée par le fait que lesdits précurseurs mélaniques sont choisis parmi la L-tyrosine, la L-dopa, le catéchol et leurs dérivés.

8. Composition selon la revendication 3, caractérisée par le fait que ladite substance naturelle est choisie parmi les cheveux humains ou l'encre de céphalopodes.

9. Composition selon l'une quelconque des revendications précédentes, caractérisée par le fait que ledit pigment mélanique est un pigment composite.

10. Composition selon la revendication précédente, caractérisée par le fait que ledit pigment composite comprend un pigment mélanique présent à la surface d'une charge particulaire, ou incorporé dans une telle charge particulaire.

11. Composition selon l'une quelconque des revendications précédentes, caractérisée par le fait que la concentration de la superoxyde dismutase est dans la gamme de 40 à 5000 unités enzymatiques de SOD, et en particulier de 300 à 1500 unités, pour 100 g de composition.

12. Composition selon l'une quelconque des revendications précédentes, caractérisée par le fait que les pigments mélaniques sont présents à raison de 0,05 à 0,5 % en poids.

13. Utilisation, en association, d'au moins une superoxyde dismutase et d'au moins un pigment mélanique comme ingrédients actifs à effet synergique dans la préparation de la composition cosmétique ou dermo-pharmaceutique destinées à lutter contre le vieillissement cutané et/ou à protéger la peau, les cheveux ou les muqueuses contre les effets nocifs et/ou inesthétiques provoqués par les radicaux libres.

14. Utilisation selon la revendication précédente, caractérisée par le fait que ladite composition est telle que définie dans l'une quelconque des revendications 1 à 12.

15. Procédé de traitement cosmétique destiné à protéger la peau et/ou les cheveux contre les effets inesthétiques provoqués par les radicaux libres et/ou à traiter ou prévenir le vieillissement précoce de la peau, caractérisé par le fait que l'on applique sur la peau et/ou les cheveux, en association, au moins une superoxyde dismutase et au moins un pigment mélanique.

16. Procédé selon la revendication précédente, caractérise par le fait que l'on applique sur la peau ou sur les cheveux une composition telle que définie dans l'une quelconque des revendications 1 à 12.

## Claims

1. Cosmetic, hygienic or dermopharmaceutical composition, characterized in that it comprises, in combination, at least one superoxide dismutase and at least one melanin pigment, the said melanin pigment being present in a proportion of 0.001 to 1% by weight.

2. Composition according to Claim 1, characterized in that the said superoxide dismutase is chosen from natural enzymes capable of catalysing the reaction of disproportionation of the superoxide ions, and any equivalent product which has such an activity.

3. Composition according to either of the preceding claims, characterized in that the said melanin pigment is chosen from:

- the products of oxidation of at least one indole or indoline compound;
- the products of polymerization of melanin precursors;
- the products resulting from the extraction of natural substances containing melanin;
- and melanin pigments obtained by culturing microorganisms.

4. Composition according to the preceding claim, characterized in that the said indole compound corresponds to the formula:

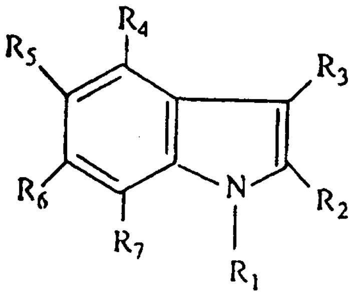

(I)

in which:

- $R_1$ and $R_3$ denote, independently of each other, a hydrogen atom or a $C_1$-$C_4$ alkyl group;
- $R_2$ denotes a hydrogen atom, a $C_1$-$C_4$ alkyl group, a carboxyl group or an alkoxy($C_1$-$C_4$)carbonyl group;
- each of the substituents $R_4$ to $R_7$ independently denotes a hydrogen atom, a $C_1$-$C_4$ alkyl group, an -NHR° or -OZ group, R° denoting a hydrogen atom, a $C_2$-$C_4$ acyl group or a $C_2$-$C_4$ hydroxyalkyl group, and Z denoting a hydrogen atom, a $C_2$-$C_{14}$ acyl group, a $C_1$-$C_4$ alkyl group or a trimethylsilyl group,
- and $R_5$ may additionally denote a halogen atom, it being understood that:
- at least one of the substituents $R_4$ to $R_7$ denotes an OZ or NHR° group, not more than one of the substituents $R_4$ to $R_7$ denoting NHR° and not more than two of the substituents $R_4$ to $R_7$ denoting OZ and, in this latter case, when Z denotes a hydrogen atom, the two substituents denoting OH are $R_5$ and $R_6$,
- and at least one of the substituents $R_4$ to $R_7$ denotes a hydrogen atom and, in the case where only one of these substituents denotes a hydrogen atom, only one of the substituents $R_4$ to $R_7$ then denotes NHR° or OZ, and the other substituents $R_4$ to $R_7$ then denote a $C_1$-$C_4$ alkyl group or else, where applicable, in the case of $R_5$, a halogen atom;

and the esters or the salts of these compounds.

5. Composition according to the preceding claim, characterized in that the said melanin pigment is obtained by oxidation of 5,6-dihydroxyindole.

6. Composition according to any one of Claims 1 to 3, characterized in that the said indoline compound corresponds to the formula:

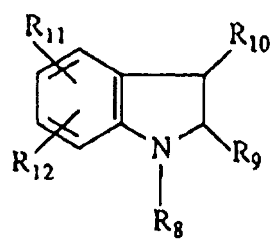

(II)

in which:

- $R_{10}$ and $R_8$ denote, independently of each other, a hydrogen atom or a $C_1$-$C_4$ alkyl radical,
- $R_9$ denotes a hydrogen atom, a $C_1$-$C_4$ alkyl radical or a carboxyl or alkoxy($C_1$-$C_4$)carbonyl group,
- $R_{12}$ denotes a hydrogen atom, a $C_1$-$C_4$ alkyl radical, hydroxyl, alkoxy ($C_1$-$C_4$), amino or $C_1$-$C_{10}$ alkylamino or halogen,
- $R_{11}$ denotes a hydrogen atom or a hydroxyl group,

on condition that at least one of the radicals $R_{11}$ and $R_{12}$ denotes a hydroxyl, alkoxy or amino group and on condition that, when $R_{11}$ denotes an amino group, $R_{12}$ cannot denote an alkylamino radical,
- it also being possible for $R_{11}$ and $R_{12}$ to form a $C_1$-$C_2$ alkylenedioxy group when they are in positions 5 and 6,

and their salts or esters.

7. Composition according to Claim 3, characterized in that the said melanin precursors are chosen from L-tyrosine, L-dopa, catechol and their derivatives.

8. Composition according to Claim 3, characterized in that the said natural substance is chosen from human hair or cephalopod ink.

9. Composition according to any one of the preceding claims, characterized in that the said melanin pigment is a composite pigment.

10. Composition according to the preceding claim, characterized in that the said composite pigment comprises a melanin pigment present at the surface of a particulate filler or incorporated in such a particulate filler.

11. Composition according to any one of the preceding claims, characterized in that the concentration of the superoxide dismutase is in the range from 40 to 5,000 SOD enzyme units and in particular from 300 to 1,500 units, per 100 g of composition.

12. Composition according to any one of the preceding claims, characterized in that the melanin pigments are present in a proportion of 0.05 to 0.5 % by weight.

13. Use, in combination, of at least one superoxide dismutase and of at least one melanin pigment as active ingredients with a synergistic effect in the preparation of the cosmetic or dermopharmaceutical composition intended for combating cutaneous aging and/or for protecting the skin, the hair or the mucosa against the harmful and/or unaesthetic effects caused by free radicals.

14. Use according to the preceding claim, characterized in that the said composition is as defined in any one of Claims 1 to 12.

15. Cosmetic treatment process intended to protect the skin and/or the hair against the unaesthetic effects caused by free radicals, and/or to treat or prevent early aging of the skin, characterized in that at least one superoxide dismutase and at least one melanin pigment are applied, in combination, to the skin and/or the hair.

16. Process according to the preceding claim, characterized in that a composition as defined in any one of Claims 1 to 12 is applied to the skin or to the hair.

**Patentansprüche**

1. Kosmetische, hygienische oder dermo-pharmazeutische Zubereitung, dadurch gekennzeichnet, daß sie zusammen mindestens eine Superoxid-Dismutase und mindestens ein Melanin-Pigment enthält, wobei das Melanin-Pigment in einer Menge von 0,001 bis 1 Gew.-% vorliegt.

2. Zubereitung nach Anspruch 1, dadurch gekennzeichnet, daß die Superoxid-Dismutase aus natürlichen Enzymen, die in der Lage sind, die Dismutationsreaktion der Superoxidionen zu katalysieren, und allen äquivalenten Produkten ausgewählt sind, die eine solche Aktivität aufweisen.

3. Zubereitung nach einem der vorhergehenden Ansprüche, dadurch gekennzeichnet, daß das Melanin-Pigment ausgewählt wird aus:

- den Oxidationsprodukten mindestens einer Indol- oder Indolinverbindung;
- den Polymerisationsprodukten der Melaninvorläufer;
- den Extraktionsprodukten natürlicher, melaninhaltiger Substanzen; und
- den Melanin-Pigmenten, die durch Mikroorganismenkulturen erhalten werden.

4. Zubereitung nach dem vorstehenden Anspruch, dadurch gekennzeichnet, daß die Indolverbindung der Formel:

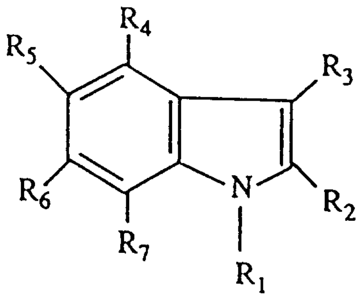

(I)

entspricht, in der:

- $R_1$ und $R_3$ unabhängig von einander ein Wasserstoffatom oder eine $C_1$-$C_4$-Alkylgruppe bedeuten;
- $R_2$ ein Wasserstoffatom, eine $C_1$-$C_4$-Alkylgruppe, eine Carboxyl- oder eine Alkoxy-($C_1$-$C_4$)-carbonylgruppe bedeuten;
- die Substituenten $R_4$ bis $R_7$ unabhängig voneinander ein Wasserstoffatom, eine $C_1$-$C_4$-Alkylgruppe, eine -NHR°- oder -OZ-Gruppe bedeuten, wobei R° ein Wasserstoffatom, eine $C_2$-$C_4$-Acylgruppe oder eine $C_2$-$C_4$-Hydroxyalkylgruppe und Z ein Wasserstoffatom, eine $C_2$-$C_{14}$-Acyl-, eine $C_1$-$C_4$-Alkyl- oder eine Trimethylsilylgruppe bedeutet, und
- $R_5$ außerdem ein Halogenatom sein kann,
- wobei mindestens einer der Substituenten $R_4$ bis $R_7$ für eine OZ- oder NHR°-Gruppe stehen, einer oder mehrere der Substituenten $R_4$ bis $R_7$ NHR° bedeuten und zwei oder mehrere der Substituenten $R_4$ bis $R_7$ OZ darstellen, und in letzterem Fall, wenn Z ein Wasserstoffatom bedeutet, die zwei OH bedeutenden Substituenten $R_5$ und $R_6$ bedeuten,
- und mindestens einer der Substituenten $R_4$ bis $R_7$ ein Wasserstoffatom bedeutet, und in dem Fall, daß ein einziger der Substituenten ein Wasserstoffatom bedeutet, einer der Substituenten $R_4$ bis $R_7$ NHR° oder OZ bedeutet und die anderen Substituenten $R_4$ bis $R_7$ dann eine $C_1$-$C_4$-Alkylgruppe bedeuten, und gegebenenfalls $R_5$ ein Halogenatom bedeutet;
  sowie die Ester oder Salze dieser Verbindungen.

**5.** Zubereitung nach dem vorstehenden Anspruch, dadurch gekennzeichnet, daß das Melanin-Pigment durch Oxidation von 5,6-Dihydroxyindol erhalten wird.

**6.** Zubereitung nach einem der vorstehenden Ansprüche 1 bis 3, dadurch gekennzeichnet, daß die Indolinverbindung der Formel

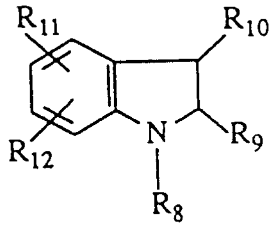

(II)

entspricht, in der:

- $R_{10}$ und $R_8$ unabhängig voneinander ein Wasserstoffatom oder einen $C_1$-$C_4$-Alkylrest bedeuten,
- $R_9$ ein Wasserstoffatom, einen $C_1$-$C_4$-Alkylrest, eine Carboxylgruppe oder ($C_1$-$C_4$)-Alkoxycarbonyl bedeuten,
- $R_{12}$ ein Wasserstoffatom, einen $C_1$-$C_4$-Alkylrest, Hydroxyl, ($C_1$-$C_4$)-Alkoxy, Amino oder $C_1$-$C_{10}$-Alkylamino oder Halogen bedeuten,
- $R_{11}$ ein Wasserstoffatom, eine Hydroxylgruppe unter der Bedingung bedeuten, daß mindestens einer der Reste $R_{11}$ und $R_{12}$ eine Hydroxyl-, Alkoxy- oder Aminogruppe bedeuten, und unter der Bedingung, daß, wenn $R_{11}$ eine Aminogruppe bedeutet, $R_{12}$ keinen Alkylaminorest bedeuten kann,

- $R_{11}$ und $R_{12}$ auch eine $C_1$-$C_2$-Alkylendioxygruppe bedeuten können, wenn sie in den Stellungen 5 und 6 vorliegen,
sowie deren Salze und Ester.

**7.** Zubereitung nach Anspruch 3, dadurch gekennzeichnet, daß die Melanin-Vorstufen unter L-Tyrosin, L-Dopa, Catechin und deren Derivate ausgewählt werden.

**8.** Zubereitung nach Anspruch 3, dadurch gekennzeichnet, daß die natürliche Substanz aus menschlichen Haaren oder Cephalopoden-Tinte ausgewählt werden.

**9.** Zubereitung nach einem der vorstehenden Ansprüche, dadurch gekennzeichnet, daß das Melanin-Pigment ein zusammengesetztes Pigment ist.

**10.** Zubereitung nach dem vorstehenden Anspruch, dadurch gekennzeichnet, daß das zusammengesetzte Pigment ein Melanin-Pigment auf der Oberfläche eines partikulären Füllstoffs oder in einen solchen partikulären Füllstoff eingearbeitetes Melanin-Pigment ist.

**11.** Zubereitung nach einem der vorstehenden Ansprüche, dadurch gekennzeichnet, daß die Konzentration der Superoxid-Dismutase im Bereich von 40 bis 5000 SOD-Enzymeinheiten, im besonderen von 300 bis 1500 Einheiten, pro 100 g der Zubereitung vorliegt.

**12.** Zubereitung nach einem der vorstehenden Ansprüche, dadurch gekennzeichnet, daß die Melanin-Pigmente im Verhältnis von 0,05 bis 0,5 Gew.-% vorliegen.

**13.** Gemeinsame Verwendung mindestens einer Superoxid-Dismutase und mindestens eines Melanin-Pigments als aktive Bestandteile mit synergistischer Wirkung bei der Herstellung einer kosmetischen oder dermo-pharmazeutischen Zubereitung zur Bekämpfung der Hautalterung und/oder zum Schutz der Haut, der Haare oder der Schleimhäute vor schädlichen und/oder unästhetischen Einflüssen, die durch freie Radikale hervorgerufen werden.

**14.** Verwendung nach dem vorstehenden Anspruch, dadurch gekennzeichnet, daß eine Zubereitung nach einem der Ansprüche 1 bis 12 verwendet wird.

**15.** Verfahren zur kosmetischen Behandlung zum Schutz der Haut und/oder der Haare gegen unästhetische Einflüsse, die durch freie Radikale hervorgerufen werden, und/oder zur Behandlung oder Vorbeugung des vorzeitigen Alterns der Haut, dadurch gekennzeichnet, daß man auf die Haut und/oder die Haare gemeinsam mindestens eine Superoxid-Dismutase und mindestens ein Melanin-Pigment aufbringt.

**16.** Verfahren nach dem vorstehenden Anspruch, dadurch gekennzeichnet, daß man auf die Haut oder auf die Haare eine Zubereitung nach einem der Ansprüche 1 bis 12 aufbringt.